# EUROPEAN PATENT APPLICATION

(11) **EP 2 571 072 A1**
(43) Date of publication of application: **20.03.2013**
(21) Application number: 11181060.2
(22) Date of filing: 13.09.2011
(51) Int. Cl.: H01L 51/00

(54) **Semiconducting thin [60]fullerene films and their use**

(71) Applicant: LANXESS Deutschland GmbH, 51369 Leverkusen (DE)
(72) Inventor: Mumyatov, Alexander V., 142432 Chernogolovka (RU); Susarova, Diana K., 142432 Chernogolovka (RU); Troshin, Pavel A., Dr., 142432 Chernogolovka (RU); Razumov, Vladimir F., Prof., 142432 Chernogolovka (RU)

(57) **Abstract**

The present invention relates to the use of soluble pentakis(alkylthio)derivatives of [60]fullerene as precursors for semiconducting thin [60]fullerene films by thermal decomposition and organic electronic devices using these films.

## Description

The present invention relates to the use of soluble pentakis(alkylthio)derivatives of [60]fullerene as precursors for semiconducting thin [60]fullerene films by thermal decomposition and organic electronic devices using these films.

Fullerene C₆₀ ([60]fullerene) is known to be one of the best organic n-type semiconductors with electron mobility, measured in oxygen and water free environment, on the order of 1 cm²/Vs [Th. B. Singh, N. Marjanovic, G. J. Matt, S. Gunes, N. S. Sariciftci, A. M. Ramil, A. Andreev, H. Sitter, R. Schwodiauer and S. Bauer, Org. Electron. 2005, 6, 105; S. Kobayashi, T. Takenobu, S. Mori, A. Fujiwara, and Y. Iwasa, Appl. Phys. Lett. 2003,.82, 4581]. Field-effect transistors based on semiconducting [60]fullerene films demonstrated exceptionally high performance. However they degrade rapidly upon exposure to ambient air as described in T. D. Anthopoulos et. al., J. Appl. Phys. 2005, 98, 054503. This problem was overcome reported by using a top protecting coating of alumina layer sputtered under Argon atmosphere. As a result the stability of Fullerene C₆₀ transistors was considerably improved and showed no degradation upon exposure to air for a period of one month [K. Horiuchi, K. Nakada, S. Uchino, S. Hashii, A. Hashimoto, N. Aoki, Y. Ochiai, and M. Shimizu, Appl. Phys. Lett. 2002, 81, 1911]. This finding has renewed interest on fullerene as a material for highly efficient field-effect transistors. As a result, a number of organic unipolar and complementary circuits have been manufactured [T. D. Anthopoulos, F. B. Kooistra, H. J. Wondergem, D. Kronholm, J. C. Hummelen, and D. M. de Leeuw, Adv. Mater. 2006, 18, 1679; B. Crone, A. Dodabalapur, Y.-Y. Lin, R. W. Fillas, Z. Bao, A. LaDuca, R. Sarpeshkar, H. E. Katz, and W. Li, Nature 2000, 403, 521; D. J. Gundlach, K. P. Pemstich, G. Wilckens, M. Grüter, S. Haas, and B. Batlogg, J. Appl. Phys. 2005, 98, 064502, T. D. Anthopoulos, B. Singh, N. Marjanovic, Niyazi S. Sariciftci, A. Montaigne Ramil, H. Sitter, M. Cölle and D. M. de Leeuw, Appl. Phys. Lett. 2006, 89, 213504].

The benefits of using semiconducting [60]fullerene thin films in organic small molecular solar cells also should not be underestimated. Single-junction photovoltaic devices with power conversion efficiencies up to 4-5% have been manufactured [N. M. Kronenberg, V. Steinmann, H. Bürckstümmer,J. Hwang, D. Hertel, F. Würthner, and K. Meerholz, Adv. Mater. 2010, 22,4193-4197; J. Wagner, M. Gruber, A. Hinderhofer, A. Wilke,B. Bröker, J. Frisch, P. Amsalem, A. Vollmer, A. Opitz, N. Koch, F. Schreiber, and W. Brütting Adv. Funct. Mater. 2010, 20, 4295-4303; R. Fitzner, E. Reinold, A. Mishra, E. Mena-Osteritz, H. Ziehlke, C. Körner, K. Leo, M. Riede, M. Weil, O. Tsaryova, A. Weiß, C. Uhrich, M.Pfeiffer, and P. Bäuerle, Adv. Funct. Mater. 2011, 21, 897-910]. A certified power conversion efficiency of more than 8% was found for multi-junction organic solar cells comprising [60]fullerene as acceptor material [http://www.heliatek.com/news-19; published by heliatec GmbH, Dresden, Germany on 2010-10-11].

Finally, it should be mentioned that [60]fullerene films are used in diode architectures that show unusual electrical properties [L. P. Ma, J. Ouyang, and Y. Yang, Appl. Phys. Lett. 2004, 84, 4786; P. Stadler, G. Hesser, T. Fromherz, G. J. Matt, H.Neugebauer, and S. N. Sariciftci, phys. stat. sol. (b) 2008, 245, 2300-2302]. In addition, thin semiconducting [60]fullerene films improved negative charge injection, reduced operation voltages and enhanced long term stability of organic light emitting diodes [J. W. Lee, J. H. Kwong, Apll. Phys. Lett. 2005, 86, 063514; X. D. Feng, C. J. Huang, V. Lui, R. S. Khangura, and Z. H. Lu. Apll. Phys. Lett. 2005, 86, 143511].

The given above examples illustrate a broad range of feasible applications for the semiconducting thin [60]fullerene films. However, industrial use of [60]fullerene is seriously limited in many cases by its poor processibility. The solubility of [60]fullerene in organic solvents is typically non-sufficient for the film preparation using printing and coating technologies. Therefore, physical vapor deposition (vacuum sublimation) is the only known technological way for growing good [60]fullerene thin films. This method has many disadvantages such as high energy consumption for creating and maintaining the vacuum conditions and heating the [60]fullerene to make it subliming and technological incompatibility with thermally-sensitive materials such as conjugated polymers that can be processed only using wet technologies. This technological incompatibility limits the area of the products where [60]fullerene can be applied. The problem was partially solved by using soluble [60]fullerene derivatives [J. L. Delgado, P.-A. Bouit, S. Filippone, M. A. Herranz, N. Martin, Chem. Commun., 2010, 46, 4853-4865]. However, derivatives of [60]fullerene are missing the excellent semiconducting properties of pristine [60]fullerene. In particular, the charge carrier mobility becomes reduced by a 2-3 orders of magnitude even for the simplest [60]fullerene derivatives [Th. B. Singh and N. S. Sariciftci, Annu. Rev. Mater. Res. 2006, 36,199-230].

It was therefore an object of the present invention to fill the gap in the fullerene technology and to provide highly soluble fullerene derivatives that can be easily manufactured and which can be used as precursors to be easily converted to the high-quality pristine [60]fullerene under mild conditions. Mild conditions in the sense of the present invention are temperatures in the range of from 70 up to 200 °C, preferably 100 up to 190 °C, very preferred 140 up to 180 °C. Highly soluble in the sense of the present invention means soluble in organic or aqueous media, preferably in alcohols or water, especially preferred in ethanol or water.

The object was achieved by the use of pentakis(alkylthio)derivatives of [60]fullerene of general formula 1 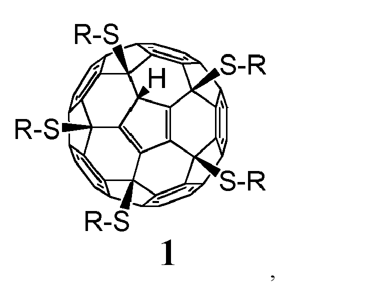 wherein R represents an optionally substituted alkyl group having 1 to 24 carbon atoms, as thermo cleavable precursors for the preparation of [60]fullerene thin films in electronic devices.

In a preferred embodiment of the present invention R represents a -( CH₂)ₙ -COOR' group,
wherein n is a number from 1 to 12 and
R' is hydrogen atom or a branched or unbranched alkyl group having 1 to 12 carbon atoms.

For clarification, it should be noted that the scope of the invention encompasses all of the definitions and parameters listed in general terms or in preferred ranges in the present specification, in any desired combination. Additionally, "[60]fullerene" represents a C₆₀ fullerene.

In a preferred embodiment of the present invention n is a number from 1 to 6. In another preferred embodiment of the present invention R'represents a branched or unbranched alkyl group having 1 to 6 carbon atoms. In a very preferred embodiment of the present invention n represents the numbers 1 or 2. In another very preferred embodiment of the present invention R' represents methyl or ethyl.

Especially preferred compounds are those ofthe formula 1a, 1b or 1c

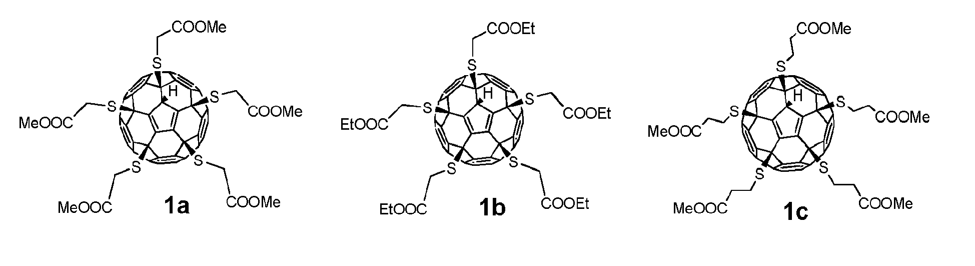

In another aspect the present invention is directed to a process for preparation of semiconducting [60]fullerene thin films by thermal decomposition of the highly soluble precursor compounds of general formula 1.

In another aspect the present invention is directed to the process for the preparation of semiconducting [60]fullerene thin films based on the [60]fullerene derivatives of general formula 1 using environmentally friendly solvents, preferably water or alcohols, especially preferred water or ethanol.

In another aspect the present invention is directed to the use of semiconducting [60]fullerene thin films grown from thermo cleavable precursors based on [60]fullerene derivatives of general formula 1 in organic field effect transistors or electronic circuits using the same.

In another aspect the present invention is directed to the use of semiconducting [60]fullerene thin films grown from thermo cleavable precursors based on [60]fullerene derivatives according to general formula 1 in photovoltaic cells.

In another aspect the present invention is directed to the use of semiconducting [60]fullerene thin films grown from thermo cleavable precursors based on [60]fullerene derivatives according to general formula 1 in organic diodes or light emitting diodes.

As described above the present invention is related to the use of pentakis(alkylthio)derivatives of [60]fullerene of general formula 1 as thermo cleavable precursors for preparation of [60]fullerene based thin films. In an embodiment of the present invention a solution of a precursor fullerene derivative according to general formula 1 in water is used for coating thin film which is produced upon annealing at elevated temperatures, preferably 70 to 200 °C, more preferred 100 to 190 °C very preferred at temperatures of from 140 to 180 °C, especially preferred at temperatures of from 135 to 150 °C forming a semiconducting film of pristine [60]fullerene. Thus the use of thermo cleavable precursors disclosed in the present invention provides an efficient way for fabrication of thin [60]fullerene films employing "wet" printing and coating technologies elaborated for highly soluble organic semiconductors. The advantage of the present invention which has been found during the experimental work is the use of environmentally friendly solvents for fabrication of [60]fullerene films.

The term "thermo cleavable precursor", as used herein, refers to a compound which decomposes at temperatures below 250 °C producing the target compound - the pristine [60] fullerene - and some volatile by-products.

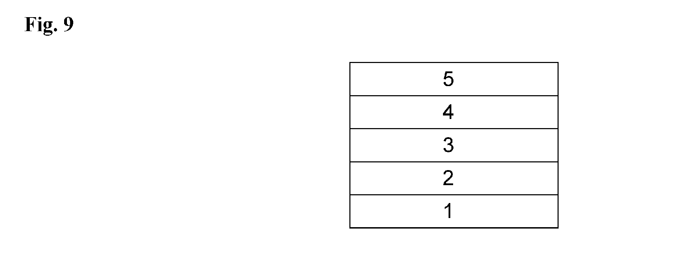

The term "photovoltaic cell", as used herein, refers to a device which generates electrical energy by absorbing light photons. According to Fig.9 such a photovoltaic cell has an indium-tin oxide bottom electrode (1) covered with a hole-selective PEDOT:PSS buffer layer (2); a poly(3-hexylthiophene)/[60]fullerene bulk heterojunction composite based on the compounds of general formula 1 as the active layer (3), calcium provided the electron-selective layer (4) and silver forms a counter electrode (5).

Such a photovoltaic cell as illustrated in Fig. 9 is constructed in the following way: The patterned ITO-coated glass substrates are sonicated consecutively in an organic solvent, preferably acetone and iso-propyl alcohol. Subsequently PEDOT:PSS is spin-coated on said glass substrates covered with an ITO layer. The resulting films are dried.

For the active layer deposition, a blend of a compound of the general formula 1 or of pristine C₆₀ and of P3HT (poly-3-hexylthiophen), both dissolved in an organic solvent, preferably chlorobenzene, is spin-coated. The resulting films are annealed and then the devices are finalized by deposition of Ca and Ag thus forming the electron-selective contact and the top electrode of the device. In a preferred embodiment of the present invention the device can be encapsulated using appropriate barrier foils and sealing adhesive materials.

In other embodiments of the present invention other materials for the bottom electrode (1), hole-selective layer (2), active layer (3), electron-selective layer (4) and top electrode (5) can be used in addition to the ones presented above or in the disclosed example. In a preferred embodiment of the present invention the active layer can comprise a composite, preferably a blend or a layer-by-layer structure, of C₆₀ prepared by decomposition of a thermo cleavable precursor based on the compounds of general formula 1 and any electron-donating organic material regardless its molecular weight and chemical composition.

Preferred electron donor materials include conjugated polymers from the group of poly(3-hexylthiophene) P3HT, poly(2,7-(9,9-di(alkyl)-fluorene)-alt-5,5-(4',7'-di-2-thienyl-2',1',3'-benzothiadiazole)) (PFDTBT), poly(2,6-(4,4-bis-(2'-ethylhexyl)-4H-cyclopenta(2,1-b;3,4,-6')dithiophene)-alt-4',7'-(2',1',3'-benzothiadiazole) (PCPDTBT), poly(2,6-(4,4-di(n-dodecyl)-4H-cyclopenta(2,1-b;3,4,-6')dithiophene)alt-5,5-(4',7'-di-2-thienyl-2',1',3'-benzothiadiazole)), poly[N-9'-heptadecanyl-2,7-carbazole-alt-5,5-(4',7'-di-2thienyl-2',1',3'-benzothiadiazole) (PCDTBT), low-molecular weight donor materials, preferably zinc or copper phthalocyanines, thiophene oligomers, organic dyes and other organic compounds characterized by their ability to form stable cationic species under chemical, photo- or electrochemical oxidation. A range of donor materials can be extended also to inorganic nanoparticles, preferably PbS, PbSe, PdTe and other colloidal nanocrystals capable of the electron donation to the appropriate acceptor compound under light irradiation.

Preferred materials to be used as hole-selective layers in photovoltaic devices are metal oxides in high valence state, preferably WO₃, MoO₃, V₂O₅, NiO, Ag₂O, and etc. At the same time, the top electrode can be composed of any metal or transparent conductive metal oxide typically applied as conductive materials in the background art.

A range of suitable n-type metal oxides such as ZnO, TiO₂, SnO₂ and some others form the electron-selective layer. Salts of alkali metals and alkali earth metals, preferably LiF, CsF, Cs₂CO₃ and etc, are also known in the background art as thin layer modifiers improving selectivity of the negative contact in the device with respect to the electron collection.

An "organic photodetector" according to an embodiment of the present invention has the same architecture as the "photovoltaic cell" described above. The procedure used for fabrication of an organic photodetector, according to an embodiment of the present invention, is essentially the same as described above. An organic photodetector is known in the background art as a device capable of the light sensing. It should be noted that in other embodiments of the present invention organic photodetectors might have different architecture, preferably possessing a lateral heterojunction instead of a bulk heterojunction and/or using other polymer-based or small molecular electron donor materials instead of P3HT and/or using other electrode materials and/or using alternative/additional buffer layers selective for specific type of charge carriers.

The term "field-effect transistor", as used herein, refers to the three-terminal electronic device where current flowing between two terminals forming channel is controlled by the voltage applied to the third terminal. An field effect transistor, according to an embodiment of the present invention, is the one having in minimal configuration
- a gate electrode composed of some metal, preferably Ca, Al, Ag or Sm or some heavily doped semiconductor, preferably Si;
- a dielectric layer composed of organic and/or inorganic materials with suitable dielectric constants;
- a thin semiconducting [60]fullerene film produced by thermal decomposition of a precursor having the general formula 1;
- source and drain electrodes composed of some metal, preferably Ca, Al, Ag or Sm or some heavily doped semiconductor, preferably Si.

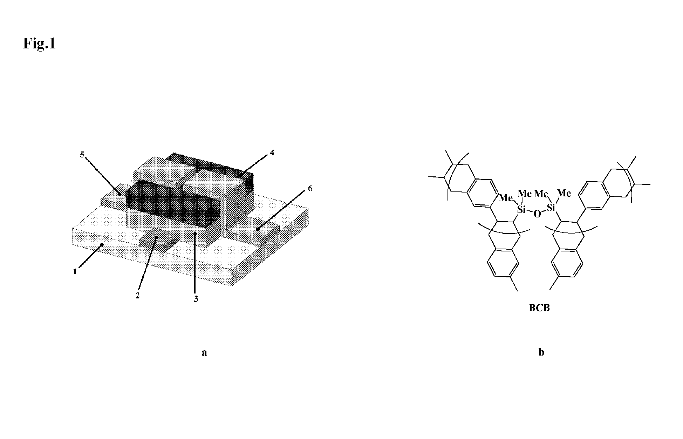

Additional layers can be optionally introduced adjacent to the gate or source/drain electrodes in order to provide rigid or flexible substrate and encapsulation protecting the device from the aggressive environment. Fig.1. is a schematic layout of a field effect transistor and molecular structure of cross-linked BCB-type dielectric according to the present invention. Such a field-effect transistor can be constructed in the following way:

The glass slides first are cleaned by sonication in base piranha solution followed by sonications in water and organic solvents, preferably acetone or iso-propanol. The cleaned slides are dried first with a stream of gas, preferably nitrogen, and then at the hot plate. These slides are used as substrates (1) for fabrication of the filed-effect transistors as shown in Fig.1. The aluminum gate electrode (2) is evaporated at the rate of 1 nm/sec to complete 200 nm thick layer. The organic benzocyclobutene-based dielectric precursor BCB (Fig.1) is spin-coated from mesitylene solution to obtain a thin film on the top of the gate electrode. The resulting precursor film was cured at elevated temperatures producing a highly cross-linked BCB layer (3). A solution of a compound of general formula 1 in an organic solvent is spin-coated on the top of the cross-linked dielectric layer. The obtained film will be decomposed at elevated temperatures in an inert atmosphere producing a crystalline thin film of pristine [60]fullerene (4). Finally, source (5) and drain (6) electrodes are evaporated in vacuum on the top of the [60]fullerene film thus producing the final device.

In other embodiments of the present invention other materials for gate (2), source (5) and drain (6) electrodes and dielectric layer (3) can be applied. In a preferred embodiment of the present invention electrodes (2), (5), (6) are composed of the same or different metals, preferably silver, gold, chromium, nickel, copper, magnesium, calcium, barium, manganese, samarium and etc. A wide range of materials can be applied for construction of dielectric layer. Considering inorganic materials one might select metal oxides selected from MgO, Al₂O₃ or SiO₂ and some other oxides with high dielectric constants. A high dielectric constant in the sense of the present invention is the constant higher than 3.9 which is characteristic for silicon dioxide. These oxides can be modified using monolayers or just thin (0.1-200 nm) layers of organic materials, preferably cross-linkable ones (like BCB) or thermally stable (such as fullerene derivatives, simple aromatic/aliphatic carboxylic or phosphonic acids and etc. In other embodiments of the present invention the dielectric layer can be made of entirely organic material such as BCB, highly functionalized fullerene derivatives, melamine, natural or synthetic hydrocarbons, amino acids or other compounds capable of functioning as dielectrics.

Spin coating is a procedure used to apply uniform thin films to flat substrates. In short, an excess amount of a solution is placed on the substrate, which is then rotated at high speed in order to spread the fluid by centrifugal force. A machine used for spin coating is called a spin coater, or simply spinner.

Rotation is continued while the fluid spins off the edges of the substrate, until the desired thickness of the film is achieved. The applied solvent is usually volatile, and simultaneously evaporates. So, the higher the angular speed of spinning, the thinner the film. The thickness of the film also depends on the concentration of the solution and the solvent.

Spin coating is widely used in microfabrication, where it can be used to create thin films with thicknesses below 10 nm. It is used intensively in photolithography, to deposit layers of photoresist about 1 micrometre thick.

Sonication is the act of applying sound-, preferably ultrasound-, energy to agitate particles in a sample, for various purposes. In the laboratory, it is usually applied using an ultrasonic bath or an ultrasonic probe, colloquially known as a sonicator. Sonication can be used to speed dissolution, by breaking intermolecular interactions. It is especially useful when it is not possible to stir the sample, as with NMR tubes. It may also be used to provide the energy for certain chemical reactions to proceed. Sonication can be used to remove dissolved gases from liquids (degassing) by sonicating the liquid while it is under a vacuum. This is an alternative to the freeze-pump-thaw and sparging methods.

Sonication can also be used to initiate crystallisation processes and even control polymorphic crystallisations. It is used to intervene in anti-solvent precipitations (crystallisation) to aid mixing and isolate small crystals.

Base piranha solution, also known as piranha etch, is a mixture of ammonia solution (NH₃H₂O) and hydrogen peroxide (H₂O₂), used to clean organic residues off substrates. Because the mixture is a strong oxidizer, it will remove most organic matter, and it will also hydroxylate most surfaces (add OH groups), making them extremely hydrophilic (water compatible).

Many different mixture ratios are commonly used, and all are called piranha. A typical mixture is 3:1 concentrated 33% ammonia to 30% hydrogen peroxide solution; other protocols may use a 4:1 or even 7:1 mixture.

Piranha solution is used frequently in the microelectronics industry, e.g. to clean photoresist residue from silicon wafers.

The term "electronic circuit", as used herein, refers to the electronic system composed of two or more electronic components appropriately connected to each other.

The term "organic diode", as used herein, refers to a two-terminal electronic device comprising organic material(s) being sandwiched between these terminals and conducting the electric current exclusively or predominantly in one direction. The term "light emitting diode", as used herein, refers to a two-terminal electronic device comprising organic material(s) sandwiched between these terminals, and capable of the light emission under applied electrical bias.

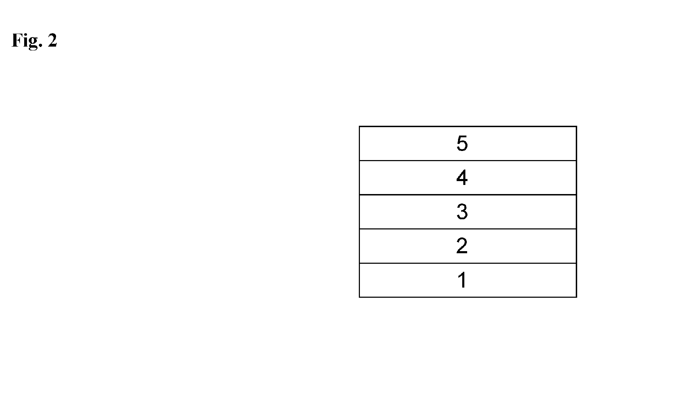

An organic diode, according to an embodiment of the present invention, is the one where in minimal configuration the semiconducting [60]fullerene film prepared from thermo cleavable precursors of general formula 1 is sandwiched between two electrodes having the same or different work functions. Additional layers can be optionally introduced adjacent to the electrodes in order to improve selectivity of the charge injection and collection or to provide a rigid or flexible substrate and/or encapsulation protecting the device from the aggressive environment. Fig.2 is a schematic layout of a organic diode architecture according to the present invention. It can be constructed in the following way: An ITO slide is cleaned by sonications in water and in organic solvents, preferably in acetone and in iso-propanol. The cleaned slide is dried with the stream of a gas, preferably nitrogen, and used as a substrate and one of the electrodes in the diode (1). To improve the surface properties of ITO (smoothness) a film of 60 nm thickness of PEDOT:PSS (2) is spin-coated on the top at 3000 rpm using a commercially available PH-type aqueous solution. The resulting film is annealed. A solution of a compound of general formula 1 in a solvent is spin-coated on the top ofthe PEDOT:PSS layer. The obtained film is decomposed in inert atmosphere producing a crystalline thin film of pristine [60]fullerene (3). Finally, the electron-injecting layer (4) composed of a low work function metal and the top electrode (5) composed of another metal are deposited in vacuum on the top of the [60]fullerene film thus producing the final diode structure. PEDOT = Poly(3,4-ethylenedioxythiophene); PSS = Poly(styrenesulfonate); ITO = Indium tin oxide. PEDOT:PSS can be purchased as a 1.3wet. % aqueous solution from Aldrich.

In other, optionally preferred embodiments of the present invention other materials for bottom (1) and top (5) electrodes, interfacial layer (2) and electron-injecting layer (4) of an organic diode can be used in addition to the ones presented above or in the example section of the present invention. A wide range of materials can be applied for construction of interfacial and electron-injecting layers. In a preferred case of a n-type diode these are n-type metal oxides such as ZnO, TiO₂, SnO₂, salts of alkali metals and alkali earth metals, preferably LiF, CsF, Cs₂CO₃ and etc and many other materials known in the background art as thin layer modifiers improving the selectivity of the negative contact in the device with respect to the electron collection.

The compounds of the general formula 1 belong already to the prior art and can be manufactured by a process described in Proceedings of the XXI Mendeleyev Competition of Students, Publication Volume, p. 55, in Russian.

The present invention is even directed to a process for raising the efficiency of electronic devices, preferably the efficiency in generating electricity from organic solar cells, wherein pentakis(alkylthio)derivatives of [60]fullerene of general formula 1 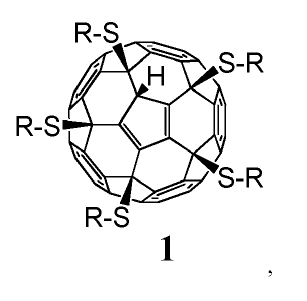 with R representing an optionally substituted alkyl group having 1 to 12 carbon atoms is used. In a further embodiment the present invention is directed to such process wherein the electronic device is a photovoltaic cell, an organic diode, a light emitting diode, an organic field effect transistor or an electronic circuit.

The present invention is even directed to a process for raising the efficiency of electronic devices characterized in that pentakis(alkylthio)derivatives of [60]fullerene of general formula 1 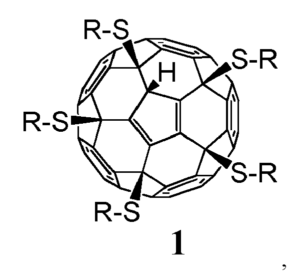 wherein R represents an optionally substituted alkyl group having 1 to 24 carbon atoms,
are used as thermo cleavable precursors for the preparation of [60]fullerene thin films.

In a preferred embodiment the present invention is directed to a process wherein a pristine [60] fullerene thin film is formed upon annealing a solution of pentakis(alkylthio)derivatives of [60]fullerene of general formula 1 in organic or aqueous media at elevated temperatures, preferably 70 to 200 °C. In a preferred embodiment the media for such process is water or alcohol., preferably water or ethanol. In a preferred embodiment the electronic device is a photovoltaic cell, an organic diode, a light emitting diode, an organic field effect transistor or an electronic circuit.

### Examples

### Example 1

### Fabrication of semiconducting [60]fullerene thin films on glass using compounds 1a-c as thermo cleavable precursors.

Thermo cleavable precursor compounds **1a, 1b and 1c** were used for the production of [60]fullerene thin films according to the present invention by dissolving them in toluene or chloroform to make solutions with material concentrations of 10-50 mg/ml.

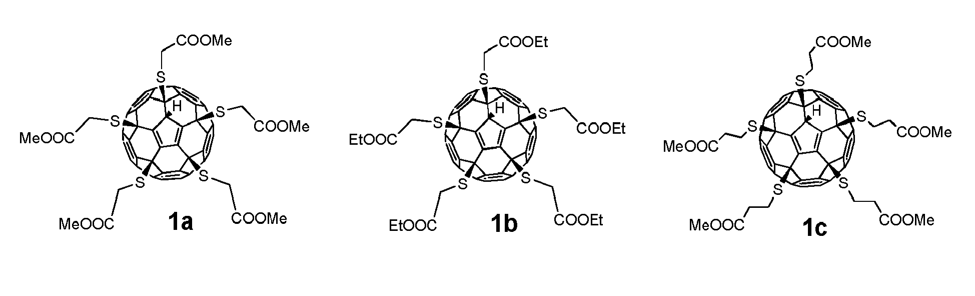

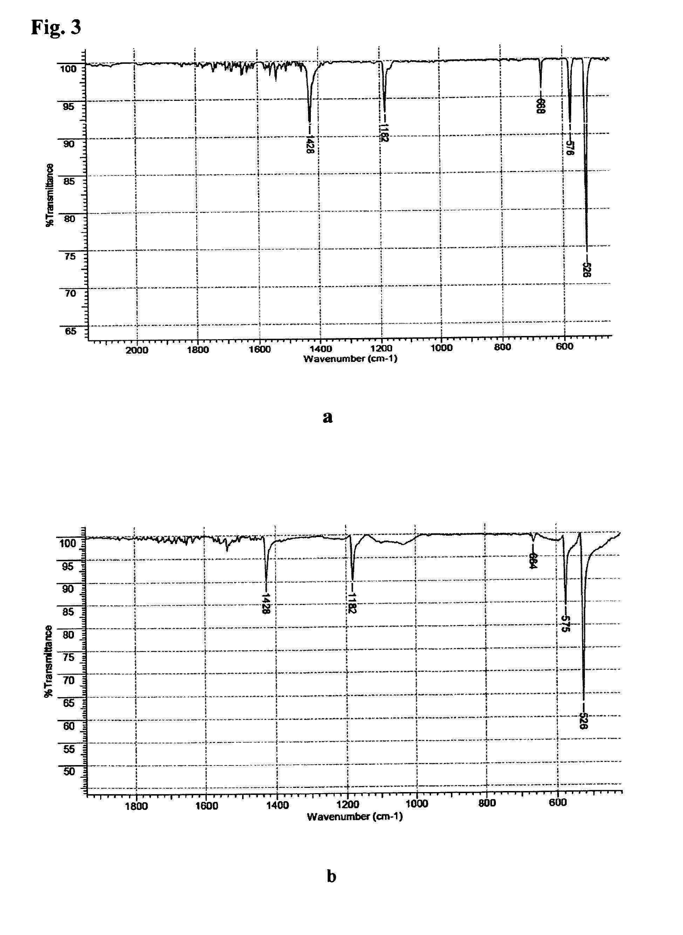

Changing the precursor concentration in the given range allows for control of the solution viscosity and the thickness of the resulting fullerene films. Vigorously cleaned 25x25 mm glass slides (base piranha solution, water, acetone, iso-propanol) were covered with the precursor solution (100 µL) and then rotated at the constant frequency of 900 rpm at the laboratory spin-coater for two minutes until it becomes completely dry. The resulting films were annealed on the hot plate installed inside argon-filled glove-box at 140 °C within 10 minutes. The films first melted and then solidified after evaporation of liquid reaction byproducts. The formed black residues were analyzed using FT-IR and UV-VIS (ultraviolet-visible) spectroscopy. An exemplary FT-IR (Fourier transform infrared spectroscopy) spectrum is shown in Fig. 3a.

All detected vibrations in the spectrum of the decomposition product correspond to the pristine [60]fullerene (Fig. 3b).

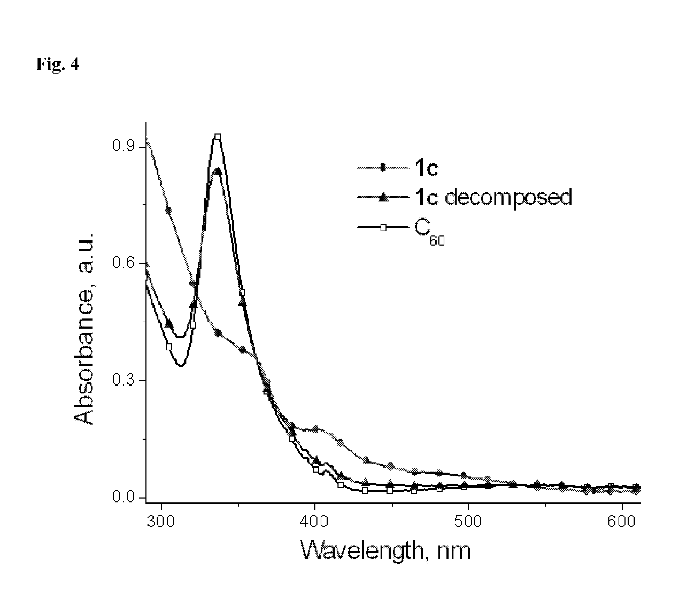

Fig.4 UV-VIS shows a spectrum of C₆₀, precursor **1c,** and the product of its thermal decomposition. The product of thermal decomposition of **1c** is (a) and the reference spectrum of pristine 99.5+% C₆₀ is (b).

The UV-VIS spectra shown in Fig.4 also supports the conclusion about the formation of pristine [60]fullerene as a product ofthe thermal decomposition of compound **1c.** It is seen from the figure in Fig.4 that all characteristic features in the spectrum of the decomposition product of **1c** closely resembles the spectrum of pristine C₆₀.

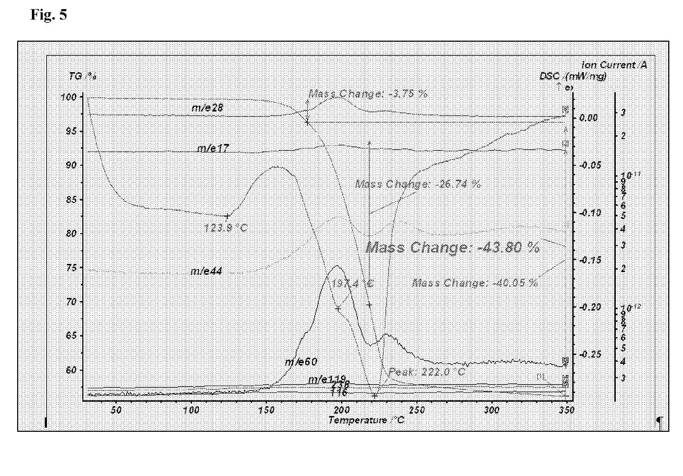

Fig.5 is the TGA profile for compound 1c. A thermal gravimetric analysis (TGA) for compound 1c revealed significant weight loss in the temperature range 150-240 °C (fig.5). The measured decrease in the sample weight corresponds well to the loss of all organic addends from the fullerene cage in the 1c molecule and the formation of parent C₆₀ as a product. The volatile products of the decomposition were analyzed by mass-spectrometry. The ions with m/z=17 (OH), 28 (CO), 44 (CO₂) 119 (-SCH₂CH₂COOMe) and 238 ([SCH₂CH₂COOMe]₂) were detected in the mass-spectra. All detected peaks can be attributed to the fragment and molecular ions corresponding to the HSCH₂CH₂COOMe and [SCH₂CH₂COOMe]₂ that are expected products of thermal decomposition of 1c.

### Example 2

Field-effect transistor based on semiconducting [60]fullerene thin films produced from thermo cleavable precursor 1c.

An exemplary field effect transistor, according is the one having in minimal a configuration of:
- a gate electrode composed of some metal such as Ca, Al, Ag or Sm or some heavily doped semiconductor such as Si;
- a dielectric layer composed of organic and/or inorganic materials with suitable dielectric constants;
- a thin semiconducting [60]fullerene film produced by thermal decomposition of a precursor having general formula 1;
- source and drain electrodes composed of some metal such as Ca, Al, Ag or Sm or some heavily doped semiconductor such as Si.

Additional layers can be optionally introduced adjacent to the gate or source/drain electrodes in order to provide rigid or flexible substrate and encapsulation protecting the device from the aggressive environment.

Such a field-effect transistor according to Fig. 1 was constructed in the following way:
The glass slides first were cleaned by sonication in base piranha solution followed by sonications in water (two times), acetone (1 time) and iso-propanol (1 time). The cleaned slides were dried first with the stream of nitrogen and then at the hot plate at 200 °C for 5 minutes. These slides were used as substrates (1) for fabrication of the filed-effect transistors (Fig.1). Aluminum gate electrode (2) is evaporated at the rate of 1 nm/sec to complete a layer of 200 nm thickness. The resulting gate electrode was immersed into a diluted solution of citric acid (150 mg per 300 mL of distilled water) and a potential of +10 V was applied for 6 minutes (stainless steel served as counter electrode). This procedure resulted in anodic passivation of the aluminum surface and growing of thin Al₂O₃ layer. Organic benzocyclobutene-based dielectric precursor BCB (Fig.1) was spin-coated from mesitylene solution (ca. 0.1%) at 900-1500 rpm to obtain 20-30 nm thick film on the top of the gate electrode. The resulting precursor film was cured at 250 °C for 12 h producing a hybrid Al₂O₃ -BCB dielectric layer (3). A solution of compound 1c in chloroform (30 mg/ml) was spin-coated on the top of the cross-linked BCB layer at 1200 rpm. The obtained film was decomposed at 180 °C within 10 minutes in inert atmosphere producing a crystalline thin film of pristine [60]fullerene (4). Finally, source (5) and drain (6) electrodes were evaporated in vacuum at10⁻⁶ mbar on the top of the [60]fullerene film thus producing the final device.

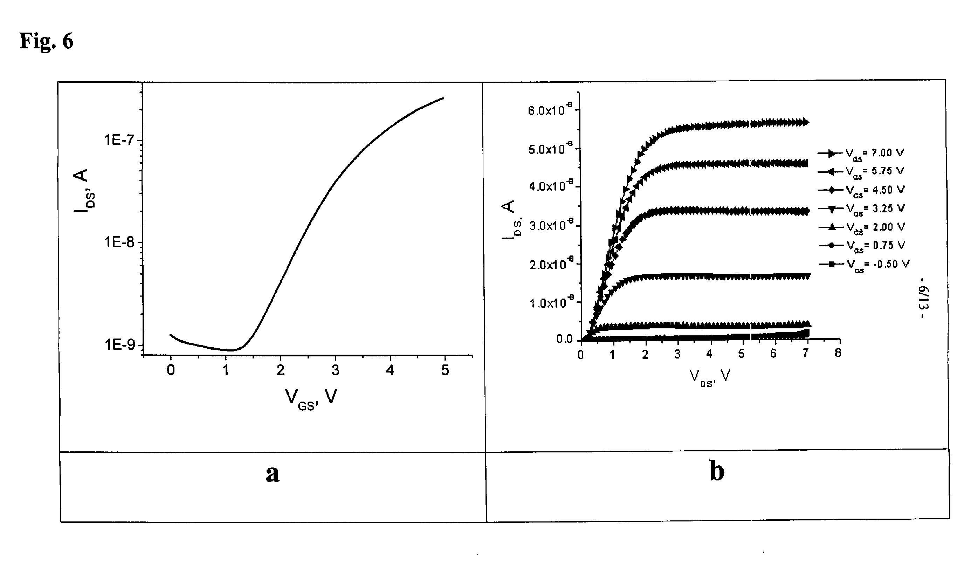

The fabricated transistor was subjected to the current-voltage measurements. First, transfer measurements were conducted. The voltage between the source and drain electrodes (V_{DS}) was set to constant, while the voltage between the source and gate (V_{GS}) was swept in appropriate range. The current flowing between the source and drain (I_{DS}) was recorded during the measurements. The obtained results are shown in Fig.6a. Fig.6a shows the logarithmic plot of the transfer current-voltage characteristic of the field-effect transistor based on the [60]fullerene films obtained by decomposition of precursor 1c.

The output measurements were conducted first by setting the voltage between the source and the gate electrodes V_{GS} to a low constant value (e.g. 0.0 V) while the voltage between the source and the drain electrodes (V_{DS}) was swept in an appropriate range (e.g. from 0 to 7 V). The current flowing between the source and the drain electrodes (I_{DS}) was recorded during the measurements. After recording the first output curve (I_{DS} vs. V_{DS}) the V_{GS} was increased to some higher value (e.g. 1.0V). One more output curve was recorded by measuring I_{DS} while sweeping V_{DS}. The V_{GS} voltage was again increased (e.g. to 2.0V) and the third output curve was measured. Typically it is sufficient to have 5-10 output curves recorded at different V_{GS} potentials to characterize the device. The exemplary set of output curves recorded for the field-effect transistor based on the [60]fullerene films obtained by decomposition of precursor 1c are shown in Fig. 6b.

It is seen from the transfer and output curves shown in Fig.6 that I_{DS} is controlled by the field created by applying the voltage V_{GS}. Thus, the fabricated device shows clear field-effect transistor behavior thus evidencing possibility for application of solution-processed precursors for deposition of semiconducting [60]fullerene films.

### Example 3

Organic diode based on semiconducting [60]fullerene thin films produced from the thermo cleavable precursor 1c.

An exemplary organic diode, according to an embodiment of the present invention, is the one where in minimal configuration the semiconducting [60]fullerene film prepared from thermo cleavable precursor 1 is sandwiched between two electrodes having the same or different work functions. Additional layers can be optionally introduced adjacent to the electrodes in order to improve selectivity of the charge injection and collection or provide rigid or flexible substrate and/or encapsulation protecting the device from the aggressive environment.

An organic diode as illustrated in Fig.2, was constructed in the following way. An ITO slide was cleaned by sonications in water (two times), acetone (1 time) and iso-propanol (1 time). The cleaned slide was dried with the stream of nitrogen and used as a substrate and one of the electrodes in the diode (1). To improve the surface properties of ITO (smoothness) a 60 nm thick film of PEDOT:PSS (2) was spin-coated on the top at 3000 rpm using commercially available PH-type aqueous solution. The resulting film was annealed at 180 °C within 15 minutes. A solution of compound 1c in chloroform (40 mg/ml) was spin-coated on the top of the PEDOT:PSS layer at 1200 rpm. The obtained film was decomposed at 180 °C within 10 minutes in inert atmosphere producing crystalline thin film of pristine [60]fullerene (3). Finally, electron-injecting layer (4) composed of calcium (20 nm) and top electrode (5) composed of silver (100 nm) were deposited in vacuum (10⁻⁶ mbar) on the top of the [60]fullerene film thus producing the final diode structure.

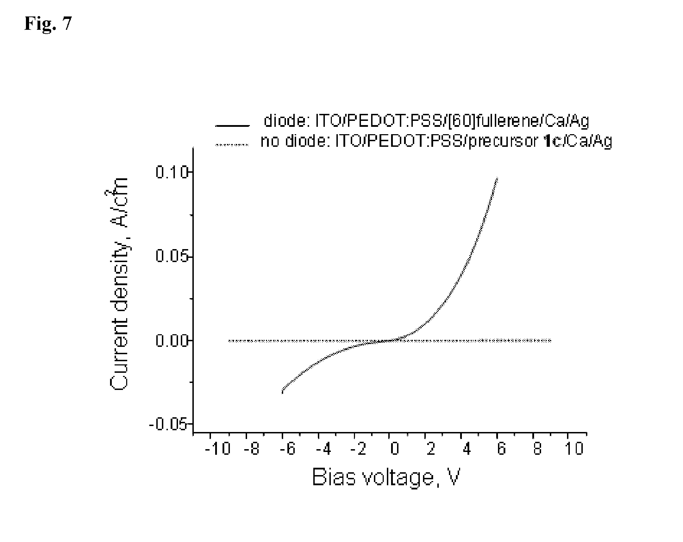

Such a manufactured organic diode was examined using I-V measurements. A bias voltage applied to the electrodes of the diode was swept between -6 and +6 V while the current flowing through the device was measured. The resulting I-V curve is shown in Fig. 7. It is seen that the device based on the [60]fullerene thin film exhibited clear diode behavior with reasonably good rectification. A reference device which comprised precursor compound 1c in the active layer showed no diode behavior even when measured between -8 and +8 V most probably due to missing semiconductor properties in the case of this compound.

### Example 4

Organic diode based on semiconducting [60]fullerene thin films produced from thermo cleavable precursor 1c and electron-selective TiO₂ buffer layer

An exemplary organic diode, according to the embodiment of the present invention, as illustrated in Fig. 2, can be constructed in the following way. An ITO slide is cleaned by sonications in water (two times), acetone (1 time) and iso-propanol (1 time). The cleaned slide is dried with the stream of nitrogen and used as a substrate and one ofthe electrodes in the diode (1). To improve the selectivity of the ITO contact a 50 nm thick film of TiO₂ (2) was deposited starting from the tetrabutyl titanate Ti(OC₄H₉)₄ through a sol-gel method reported in Appl. Phys. Lett. 2008, 93, 193307. The procedure for the preparation of TiO₂-sol involved the dissolution of 10 mL of Ti(OC₄H₉)₄ in 60 mL of ethanol C₂H₅OH followed by the addition of 5 ml of acetyl acetone. Then a solution composed of 30 ml of C₂H₅OH, 10 ml of de-ionized water, and 2 ml of hydrochloric acid (HCl) with the concentration of 0.28 mol/L was added dropwise under vigorous stirring. The resulting mixture was stirred at room temperature for additional 2 h.

The prepared TiO₂-sol solution was spin-coated on the cleaned ITO-coated glass substrates at 3000 rpm. The resulting films were dried in air for 20 min and then were transferred to the chamber oven heated up to 450 °C (means that the samples were brought into the hot oven). The annealing at 450 °C takes typically 2 hrs. Annealed TiO₂ slides were ready for coating of the next layer. A solution of compound 1c in chloroform (40 mg/ml) was spin-coated on the top of the TiO₂ layer at 1200 rpm. The obtained film was decomposed at 180 °C within 10 minutes in inert atmosphere producing crystalline thin film of pristine [60]fullerene (3). Finally, electron-injecting layer (4) composed of calcium (20 nm) and top electrode (5) composed of silver (100 nm) were deposited in vacuum (10⁻⁶ mbar) on the top of the [60]fullerene film thus producing the final diode structure.

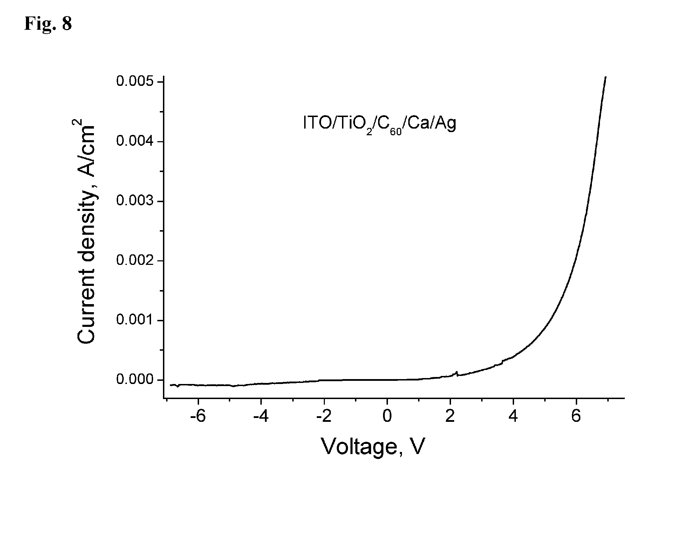

The diode prepared in such way was examined using I-V measurements as described above (Example 3). Fig. 8 shows current-voltage characteristics of an organic diode using TiO₂ electron injection layer and a [60]fullerene film produced by thermal decomposition of 1c.The resulting I-V curve shown in Fig. 8 demonstrates a clear diode behavior of the device.

The presented example illustrates that thin films of [60]fullerene produced by decomposition of precursor compounds of general formula 1 can be successfully applied for construction of organic n-type diodes.

### Example 5

Bulk heterojunction photovoltaic cell utilizing [60]fullerene produced by decomposition of the thermo cleavable precursor 1c.

For this experiment a photovoltaic cell with indium-tin oxide was used as bottom electrode (1) covered with a hole-selective PEDOT:PSS buffer layer (2); poly(3-hexylthiophene)/[60]fullerene bulk heterojunction composite as the active layer (3), 20 nm of calcium provided the electron-selective layer (4) and 100nm of silver (100 nm) formed a counter electrode (5).

Such a photovoltaic cell as illustrated in Fig. 9 was constructed in the following way: The patterned ITO-coated glass substrates were sonicated consecutively with acetone and iso-propyl alcohol for 10 min. Subsequently PEDOT:PSS (Baytron® PH, now even available as Clevios ™ PH from HC Starck Clevios GmbH, Leverkusen, Germany) was spin-coated on glass substrates covered with ITO layer at 3000 rpm. The resulting films were dried in air for 20 min at 180 °C. Fig.9 is a schematic layout of the claimed bulk heterojunction photovoltaic cell architecture according to the present invention as used in the present example.

For the active layer deposition, a blend of 11 mg of 1c (or 6.0 mg of pristine C₆₀) and 12 mg of P3HT, (poly-3-hexylthiophen) both dissolved in 1 mL of chlorobenzene, was spin-coated at the spinning frequency of 900 rpm. The resulting films were annealed at 200 °C for 7 min and then the devices were finalized by deposition of 20 nm of Ca and 100 nm of Ag thus forming electron-selective contact and the top electrode of the device. The device can be encapsulated using appropriate barrier foils and sealing adhesive materials.

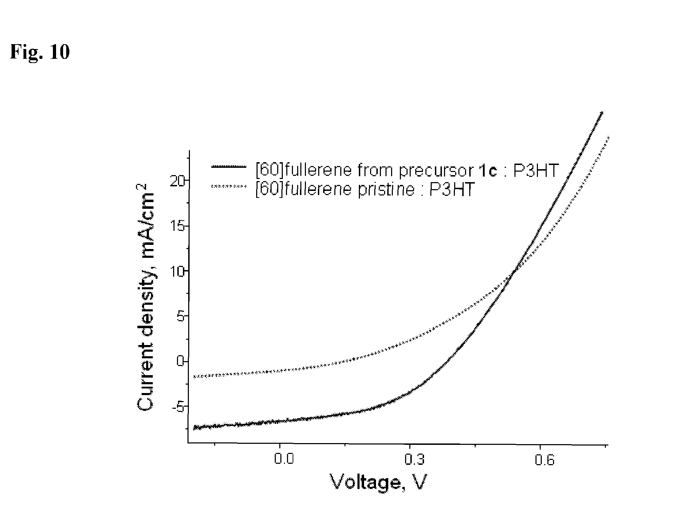

The photovoltaic operation of the fabricated deviceas described above was revealed using current density - voltage (I-V) measurements performed under standard solar cell testing conditions. A KHS Steuernagel (Solar Cell Test 575) solar simulator was used as a light source providing AM1.5 irradiation of 100 mW/cm² intensity while the cell was kept at 25 °C. The I-V curves measured for two different [60]fullerene:P3HT devices are shown in Fig.10. The first device comprised a blend of P3HT and [60]fullerene produced by decomposition of precursor compound 1c. The second device was a reference where P3HT (12 mg) was blended in the chlorobenzene solution (1 mL) with pristine [60]fullerene (6.0 mg). The solubility of C₆₀ in chlorobenzene approaches 8 mg/ml, therefore concentration of 6.0 mg/ml could be easily reached. Fig. 10. shows the result in I-V curves obtained for bulk heterojunction solar cells based on [60]fullerene:P3HT composites.

It is seen from Fig.10 that a photovoltaic cell based on P3HT:pristine [60]fullerene composite shows very weak photovoltaic performance. The following parameters were obtained: short circuit current density I_{sc}=1.0 mA/cm²; open circuit voltage V_{oc}=141 mV; fill factor FF=30% and light power conversion efficiency η=0.04%. On the contrary, the exemplary photovoltaic cell utilizing P3HT and [60]fullerene produced from thermo cleavable precursor 1c according to the embodiment of the present invention showed significantly higher performance: I_{sc}=5.4 mA/cm² (improvement by a factor of 5), V_{oc}=531 mV (improvement by a factor of∼4), FF=42% and η=1.2% (improvement by a factor of ∼30).

The presented results prove that generation of C₆₀ *in-situ* in the polymer matrix by decomposing of thermo cleavable precursor might be considered as a promising approach for construction of efficient organic photovoltaic devices.

### Example 6

Organic photodetector utilizing [60]fullerene produced by decomposition of thermo cleavable precursor 1c.

An organic photodetector as used in the present example had the same architecture as the photovoltaic cell described in Example 5. The procedure used for fabrication of such an organic photodetector was essentially the same as described above in example 5.

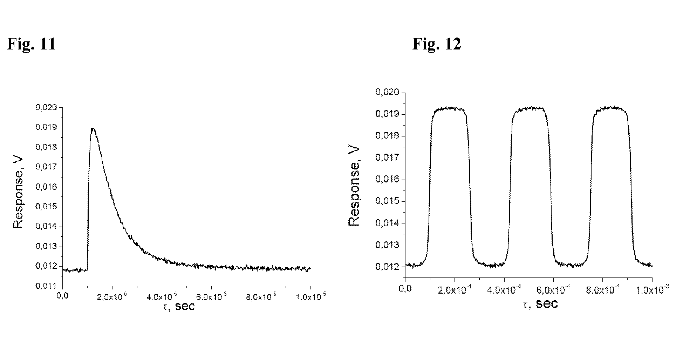

To illustrate this ability for an organic photodetector according to the present invention, the following experiment was performed. The device electrodes were connected to the oscilloscope which was used to monitor the evolution of the electrical potential at the electrodes as a function of time. First, short laser pulse (nitrogen laser, 337 nm, 10 ns) was applied to the device. Absorption of the light generated electrical charges that induced photovoltage at the electrodes of the device which was detected by the oscilloscope (Fig. 11). The decay of this signal occurs within few microseconds. Therefore, the exemplary photodetector device potentially could detect 10⁴ - 10⁵ short light signals per second which demonstrates a potential for its high frequency operation. The result of the experiment is given in Fig.11 showing a transient photoresponse of the organic photodetector based on the [60]fullerene:P3HT composite prepared according to the present invention. As shown in Fig.12 the organic photodetector according to the present invention revealed an excellent detection of a modulated light signal.

### Example 7

Lateral (planar) heterojunction photovoltaic cell utilizing [60]fullerene produced by decomposition of a thermo cleavable precursor 1c.

An exemplary photovoltaic cell as used in the present example is the one where indium-tin oxide is used as bottom electrode (1) covered with a hole-selective PEDOT:PSS buffer layer (2); poly(3-hexylthiophene) is used as bottom p-type electron donor layer (3), [60]fullerene produced from thermo cleavable precursor 1c forms the upper n-type electron accepting layer (4). Thin (20 nm) layer of calcium provides electron-selective contact (5) and 100nm of silver (100 nm) forms a counter electrode (6).

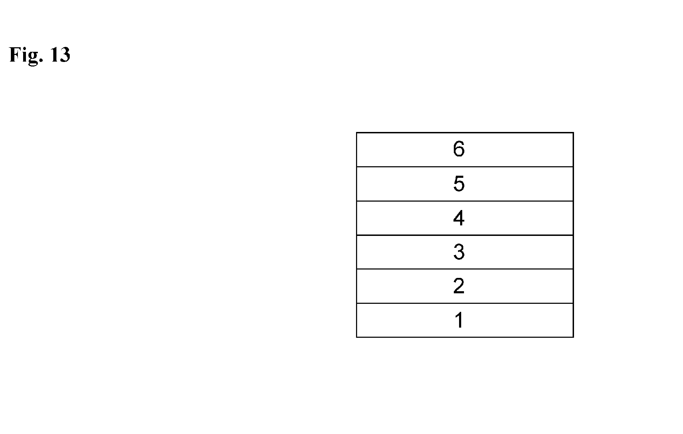

The photovoltaic cell, according to the present example of lateral (planar) heterojunction photovoltaic cell architecture is illustrated in Fig.13 and was constructed in the following way: The patterned ITO-coated glass substrates were sonicated consecutively with acetone and iso-propyl alcohol for 10 min. Subsequently PEDOT:PSS (Baytron® PH, now even available as Clevios™ PH from HC Starck Clevios GmbH, Leverkusen, Germany) was spin-coated on glass substrates covered with ITO layer at 3000 rpm. The resulting films were dried in air for 20 min at 180 °C.

The solution of 18 mg of P3HT in 1 mL of chlorobenzene was spin-coated at the spinning frequency of 1200 rpm on the top of PEDOT:PSS film. The resulting films were annealed at 140 °C for 10 min. Afterwards, a solution of 15 mg 1c in 1 ml of tetrahydrofurane (THF) was spin-coated on the top of the P3HT layer at the spinning frequency of 2500 rpm. The resulting sandwich structure was dried in vacuum and then annealed at 180 °C for 5 min. The cell was finalized by deposition of 20 nm of Ca and 100 nm of Ag thus forming electron-selective contact and the top electrode of the device. The device can be encapsulated using appropriate barrier foils and sealing adhesive materials.

A reference lateral heterojunction device was fabricated for comparison. The annealed P3HT films were produced identically as described in the above procedure and were assembled in a vacuum chamber and [60]fullerene was evaporated on the top thus creating 30 nm thick layer. The resulting sandwich structure was annealed at 180 °C for 5 min. The cell was finalized by deposition of 20 nm of Ca and 100 nm of Ag thus forming electron-selective contact and the top electrode of the device.

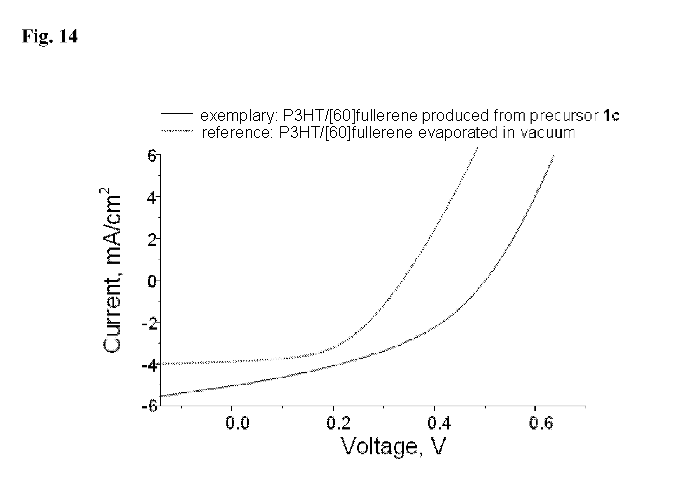

The photovoltaic operation of the fabricated exemplary lateral heterojunction device comprising [60]fullerene layer produced by decomposition of precursor compound 1c was compared to the reference device comprising [60]fullerene layer which was thermally evaporated in vacuum. The current density - voltage (I-V) measurements were performed under standard solar cell testing conditions (AM1.5, 100 mW/cm², 25°C). The solar simulator KHS Steuernagel (Solar Cell Test 575) was used as a light source providing AM1.5 irradiation of 100 mW/cm² intensity while the cell was kept at 25 °C. The obtained I-V curves measured for the exemplary and the reference [60]fullerene/P3HT devices with lateral heterojunction are shown in Fig.14.

As can be seen in Fig.14 the reference lateral heterojunction cell showed reasonable photovoltaic performance. The following parameters were obtained: short circuit current density I_{sc}=3.2 mA/cm²; open circuit voltage V_{oc}=334 mV; fill factor FF=47% and light power conversion efficiency η=0.5%. The exemplary cell with lateral heterojunction based on P3HT covered with the [60]fullerene layer produced from precursor 1c showed two times higher overall performance and improvement in all parameters with except for the fill factor: I_{sc}=5.0 mA/cm² (improvement by 56%), V_{oc}=495 mV (improvement by 48%); fill factor FF=39% and light power conversion efficiency η=1.0% (improvement by a factor of 2).

Therefore, the application of thermo cleavable precursor compounds of general formula 1 allows designing planar heterojunction photovoltaic architectures without the use of expensive vacuum evaporation processes. Moreover, the formation of the [60]fullerene layer by decomposition of solution-processible precursors 1 seems to be even beneficent for the photovoltaic device operation compared to the growth of the [60[fullerene layers from the vapor phase.

## Claims

1. Use of pentakis(alkylthio)derivatives of [60]fullerene of general formula 1 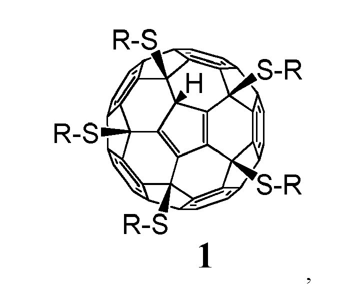 wherein R represents an optionally substituted alkyl group having 1 to 24 carbon atoms,
as thermo cleavable precursors for the preparation of [60]fullerene thin films in electronic devices.

2. Use according to Claim 1 **characterized in that** R represents the -( CH₂)ₙ -COOR'group wherein n is a number from 1 to 12 and R' represents a hydrogen atom or a branched or unbranched alkyl group having 1 to 12 carbon atoms.

3. Use according to Claim 2 **characterized in that** n is a number from 1 to 6.

4. Use according to Claims 2 to 3 **characterized in that** R'represents a branched or unbranched alkyl group having 1 to 6 carbon atoms.

5. Use according to Claims 2 to 4 **characterized in that** n represents the numbers 1 or 2.

6. Use according to Claims 2 to 5 **characterized in that** R' represents methyl or ethyl.

7. Use according to Claims 1 to 6 **characterized in that** the pentakis(alkylthio)derivatives of [60]fullerene are those of the formula 1a, 1b or 1c

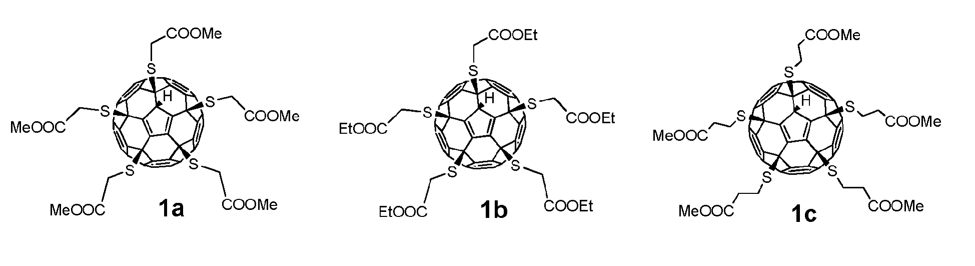

8. Use according to Claims 1 to 7, **characterized in that** the organic electronic devices are photovoltaic cells, organic diodes, light emitting diodes, organic field effect transistors or electronic circuits using the same.

9. Use according to Claims 1 to 7 **characterized in that** the [60]fullerene thin film is grown from thermo cleavable precursors based on the [60]fullerene derivatives of formula 1 by thermal decomposition.

10. Use according to Claim 9 **characterized in that** environmentally friendly solvents are used for the manufacture of the [60]fullerene thin film, preferably water or alcohols, very preferred water or ethanol.

11. Use according to Claims 9 or 10 **characterized in that** the thermal decomposition results in pristine [60]fullerene.

12. A process for raising the efficiency of electronic devices **characterized in that** pentakis(alkylthio)derivatives of [60]fullerene of general formula 1 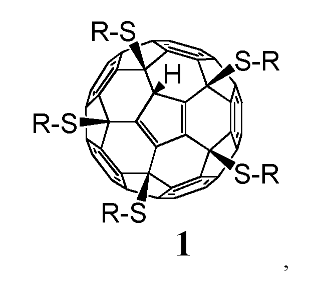 wherein R represents an optionally substituted alkyl group having 1 to 24 carbon atoms,
are used as thermo cleavable precursors for the preparation of [60]fullerene thin films.

13. A process according to Claim 12 **characterized in that** a pristine [60] fullerene thin film is formed upon annealing a solution of pentakis(alkylthio)derivatives of [60]fullerene of general formula 1 in organic or aqueous media at elevated temperatures, preferably 70 to 200 °C.

14. A process according to Claim 13 **characterized in that** the media is water or alcohol, preferably water or ethanol.

15. A process according to Claims 12 or 13, **characterized in that** the electronic device is a photovoltaic cell, an organic diode, a light emitting diode, an organic field effect transistor or an electronic circuit.
